# EUROPEAN PATENT SPECIFICATION

(11) **EP 4 308 925 B1**
(45) Date of publication and mention of the grant of the patent: **05.08.2026**
(21) Application number: 22712935.0
(22) Date of filing: 14.03.2022
(51) Int. Cl.: G01N 33/50, C12N 15/62, G01N 33/58

(54) **METHOD FOR SCREENING POTENTIAL THERAPEUTIC AGENTS**
VERFAHREN ZUM SCREENING POTENZIELLER THERAPEUTISCHER MITTEL
PROCÉDÉ DE CRIBLAGE D'AGENTS THÉRAPEUTIQUES POTENTIELS

(30) Priority: 15.03.2021 EP 21162636
(43) Date of publication of application: 24.01.2024
(73) Proprietor: Universiteit Antwerpen, 2000 Antwerpen (BE)
(72) Inventor: DEBEN, Christophe, 2550 Kontich (BE); CARDENAS, Edgar, 2000 Antwerpen (BE); LE COMPTE, Maxim, 2620 Hemiksem (BE); LIN, Abraham, 2000 Antwerpen (BE)
(74) Representative: De Clercq & Partners
(86) International application number: PCT/EP2022/056501
(87) International publication number: WO 2022/194760

(56) References cited:
- WO-A1-2019/122388
- DEBEN CHRISTOPHE ET AL: "OrBITS: A High-throughput, time-lapse, and label-free drug screening platform for patient-derived 3D organoids", BIORXIV, 11 September 2021 (2021-09-11), XP55914940, Retrieved from the Internet <URL:https://www.biorxiv.org/content/10.1101/2021.09.09.459656v1.full.pdf> [retrieved on 20220425], DOI: 10.1101/2021.09.09.459656
- MASON J. ET AL: "High throughput screen identifies stroma modulatory drugs that inhibit PDAC progression", vol. 20, 1 November 2020 (2020-11-01), CH, pages S125 - S126, XP055823209, ISSN: 1424-3903, Retrieved from the Internet <URL:http://dx.doi.org/10.1016/j.pan.2020.07.229> DOI: 10.1016/j.pan.2020.07.229
- CATTANEO CHIARA M ET AL: "Tumor organoid-T-cell coculture systems", NATURE PROTOCOLS, NATURE PUBLISHING GROUP, GB, vol. 15, no. 1, 18 December 2019 (2019-12-18), pages 15 - 39, XP036977352, ISSN: 1754-2189, [retrieved on 20191218], DOI: 10.1038/S41596-019-0232-9
- BODE KONSTANTIN J ET AL: "A fast and simple fluorometric method to detect cell death in 3D intestinal organoids", vol. 67, no. 1, 1 July 2019 (2019-07-01), US, pages 23 - 28, XP055823175, ISSN: 0736-6205, Retrieved from the Internet <URL:https://www.future-science.com/doi/pdfplus/10.2144/btn-2019-0023> DOI: 10.2144/btn-2019-0023
- XIN XIN ET AL: "Development of a dual fluorescence system for simultaneous detection of two cell populations in a 3D coculture", PROCESS BIOCHEMISTRY, ELSEVIER LTD, GB, vol. 86, 23 July 2019 (2019-07-23), pages 144 - 150, XP085858727, ISSN: 1359-5113, [retrieved on 20190723], DOI: 10.1016/J.PROCBIO.2019.07.018
- GASPAR NATASA ET AL: "NanoBiT System and Hydrofurimazine for Optimized Detection of Viral Infection in Mice-A Novel in Vivo Imaging Platform", INTERNATIONAL JOURNAL OF MOLECULAR SCIENCES, vol. 21, no. 16, 15 August 2020 (2020-08-15), pages 5863, XP055823112, DOI: 10.3390/ijms21165863
- WANG Y ET AL: "RENILLA LUCIFERASE-AEQUOREA GFP (RUC-GFP) FUSION PROTEIN, A NOVEL DUAL REPORTER FOR REAL-TIME IMAGING OF GENE EXPRESSION IN CELL CULTURES AND IN LIVE ANIMALS", MGG - MOLECULAR GENETICS AND GENOMICS, SPRINGER, BERLIN, DE, vol. 268, no. 2, 1 October 2002 (2002-10-01), pages 160 - 168, XP002977220, ISSN: 1617-4615, DOI: 10.1007/S00438-002-0751-9
- DEBEN CHRISTOPHE ET AL: "OrBITS: A High-throughput, time-lapse, and label-free drug screening platform for patient-derived 3D organoids", BIORXIV, 11 September 2021 (2021-09-11), XP055914940, Retrieved from the Internet <URL:https://www.biorxiv.org/content/10.1101/2021.09.09.459656v1.full.pdf> [retrieved on 20220425], DOI: 10.1101/2021.09.09.459656

## Description

### Field of the invention

The present invention is in a field of high-throughput screening of potential therapeutic agents against a co-culture of cellular objects.

### Background to the invention

Patient-derived cellular objects, in particular patient derived tumour organoids (PDOs) are considered patients-in-the-lab, since the patient's tumour can be grown with high efficiency. An increasing amount of evidence suggest that these PDOs can predict therapy response in the patient following *in vitro* drug screening. The current gold-standard assay is based on the measurement of intracellular ATP levels as an endpoint assay using the CellTiter-Glo 3D assay (Promega). This assay requires cell lysis, is limited to one time-point, and cannot distinguish cytostatic (*i.e.* growth arrest) from a cytotoxic *(i.e.* cell death) response. Moreover, within a co-culture containing PDO and another cell type (e.g. fibroblasts, immune cell(s) (PBMC, NK-cell, Cytotoxic T-cell,...) or endothelial cells) the assay cannot distinguish cell type specific responses within the co-culture. Mason et al in "High throughput screen identifies stroma modulatory drugs that inhibit PDAC progression" Pancreatology, vol. 20, 1 November 2020, pages S125-S126, discloses a screening method that identifies stroma modulatory drugs that inhibit PDAC progression, wherein two cell types, PDAC organoids and PSCs, are modified to express fluorescent proteins particular to each which are subsequently imaged, however, the method cannot distinguish a cytostatic from a cytotoxic response. Deben Christophe et al: "OrBITS: A High-throughput, time-lapse, and label-free drug screening platform for patient-derived 3D organoids", bioRxiv, 11 September 2021 discloses a combination of bright-field imaging and fluorescent death markers to differentiate between cytotoxic and cytostatic effects of drugs in co-cultures of different lung cancer cell lines. The methods described herein overcome the problems of the art.

### Summary of the invention

Provided is a method for determining an effect of a potential active agent on a sample according to claim 1, comprising a co-culture comprising a target cellular object, TCO, and one or more further cell types, FCT, wherein:
- the TCO has been labelled with a fluorescent live-cell marker having a first emission/excitation profile, and with a luminescent cell-death marker, and
- optionally the FCT has been labelled with a fluorescent live-cell marker having a second emission/excitation profile different from the first emission/excitation profile,
comprising the steps:
- capturing, during an acquisition event using a microscope, a dataset comprising:
   - a first fluorescent image from the fluorescent live-cell marker having the first emission/excitation profile, and
   - optionally a second fluorescent image from the fluorescent live-cell marker having the second emission/excitation profile, and
   - a luminescence measurement of the sample,
   wherein a plurality of datasets is captured, of which one or a plurality of test datasets is captured for a sample contacted with the potential active agent and one or a plurality of control datasets is captured for a sample not contacted with the potential active agent,
- determining a growth of the TCO and cell death of the TCO, and optionally growth of the FCT independent of the growth of the TCO, wherein:
   - growth of the TCO is determined from:
      - an area of the TCO determined from the first fluorescent image captured in each dataset at different time points, or
      - an intensity of the TCO determined from the first and second fluorescent images respectively captured in each dataset at different time points, or
      - a combination of area and intensity of the TCO determined from the first fluorescent images captured in each dataset at different time points, and
   - cell death of the TCO is determined from the luminescence captured in each dataset at the different time points
   - optionally growth of the FCT is determined from:
      - an area of the FCT determined from the second fluorescent images captured in each dataset, or
      - an intensity of the FCT determined from the second fluorescent images captured in each dataset, or
      - a combination of area and intensity of the FCT determined from the second fluorescent image captured in each dataset,
wherein:
- the effect of the potential active agent is determined from a difference, between test datasets and control datasets, in growth for the TCO and in cell death of the TCO, and optionally in growth for the FCT,
- a difference that is a decrease in growth in the absence of cell death is indicative that potential active agent has a cytostatic effect, and
- a difference that is a decrease in growth and increase in cell death is indicative that potential active agent has a cytotoxic effect, and
wherein:
- the TCO is a patient-derived organoid or cancer cell line derived spheroid, and
- the FCT is a fibroblast(s) or an immune cell(s) such as PBMC, NK-cell, Cytotoxic T-cell, or an endothelial cell(s),
   or
- the TCO is a fibroblast(s) or an immune cell(s) such as PBMC, NK-cell, Cytotoxic T-cell, or an endothelial cell(s), and
- the FCT is a patient-derived organoid or cancer cell line derived spheroid,
thereby establishing both cytostatic effect and cytotoxic effect of the potential active agent.

The method may further comprise steps of determining interactions between the TCO and FCT as independent objects,
wherein interactions is determined from:
   - a merging between the TCO and the FCT determined from the first and second fluorescent images respectively captured in each dataset at the different time points, and/or
   - a splitting of the TCO and the FCT associated determined from the first and second fluorescent images respectively captured in each dataset at the different time points, and
the effect of the potential active agent is determined from a difference, between test datasets and control datasets, in the interactions.

The method may further comprise steps of determining activity of the TCO and FCT as independent objects,
wherein activity is determined from:
   - a velocity of the TCO determined from the first fluorescent images captured in each dataset at (the) different time points, and
   - a velocity of the FCT determined from the second fluorescent images captured in each dataset at (the) different time points, and
the effect of the potential active agent is determined from a difference, between test datasets and control datasets, in the activity.

The method may further comprise steps of determining activity of the TCO and optionally activity of the FCT as object independent of the TCO, wherein
- activity of the TCO is determined from a velocity of the TCO determined from the first fluorescent images captured in each dataset at different time points, and
- optionally activity of the FCO is determined from a velocity of the FCT determined from the second fluorescent images captured in each dataset at different time points, and
the effect of the potential active agent is determined from a difference, between test datasets and control datasets, in the activity.

The method may further comprise a step of determining an area of the TCO, or of the FCT, or of a combined TCO-FCT object from the fluorescence images wherein the effect of the potential active agent is determined from a difference in area of the TCO, or of the FCT, or of the combined TCO-FCT object between the test dataset and control dataset.

The method may further comprise a step of determining an intensity of the TCO, or of the FCT, or of a combined TCO-FCT object from the fluorescence images wherein the effect of the potential active agent is determined from a difference in intensity of the TCO, or of the FCT, or of the combined TCO-FCT object between the test dataset and control dataset.

The method may further comprise a step of determining a velocity of the TCO, or of the FCT, or of a combined TCO-FCT object from the fluorescence images wherein the effect of the potential active agent is determined from a difference in velocity of the TCO, or of the FCT, or of the combined TCO-FCT object between the test dataset and control dataset.

The method may further comprise a step of determining a contractility of the TCO, or of the FCT, or of a combined TCO-FCT object from the fluorescence images wherein the effect of the potential active agent is determined from a difference in contractility of the TCO, or of the FCT, or of the combined TCO-FCT object between the test dataset and control dataset.

The method may further comprise a step of determining a splitting of the TCO, or of the FCT, or of a combined TCO-FCT object from the fluorescence images wherein the effect of the potential active agent is determined from a difference in splitting of the TCO, or of the FCT, or of the combined TCO-FCT object between the test dataset and control dataset.

The method may further comprise a step of determining a merging of the TCO, or of the FCT, or of a combined TCO-FCT object from the fluorescence images wherein the effect of the potential active agent is determined from a difference in merging of the TCO, or of the FCT, or of the combined TCO-FCT object between the test dataset and control dataset.

The cell death of the TCO may be determined, for each dataset, from the luminescence captured and from a quantity of cells present in the TCO measured from the first fluorescent image.

Further provided, but not currently claimed, is a fluorescent-luminescent fusion protein comprising a live-cell fluorescent label (protein), a luminescent cell-death marker (protein) linked by a self-cleaving peptide, wherein:
- the self-cleaving peptide is configured to be cleaved within the cell to separate the expressed live-cell fluorescent label and luminescent cell-death marker;
- the luminescent cell-death marker produces luminescence when the cell wall is breached (e.g. apoptosis, lysis, cell death) by reaction with one or more reagents in a cell culture medium;
- the live-cell fluorescent label is fluorescently active at least within the cell.

Further provided, but not currently claimed, is a genetic construct encoding the fusion protein described herein. Further provided is an expression vector comprising the genetic construct described herein.

### Figure Legends

**FIG. 1A** depicts a first fluorescent image of a TCO that is an organoid superimposed upon a second fluorescent image of associated FCTs that are fibroblasts; **FIGs. 1****B** and **1C** show separately the first and second fluorescent images respectively.
**FIG. 2A** depicts a first fluorescent image of an TCO that is an organoid that is splitting superimposed upon a second fluorescent image of associated FCTs that are fibroblasts; **FIGs. 2B** and **2C** show separately the first and second fluorescent images respectively.
**FIG. 3A** depicts a first fluorescent image of TCOs that are organoids advancing towards each other superimposed upon a second fluorescent image of associated FCTs that are fibroblasts arranged as a contracting ring (contractility); **FIGs. 3B** and **3C** show separately the first and second fluorescent images respectively.
**FIG. 4A** depicts a first fluorescent image of a TCO that is an organoid superimposed upon a second fluorescent image of associated FCTs that are fibroblasts moving towards the organoids; **FIGs. 4B** and **4C** show separately the first and second fluorescent images respectively.
**FIG. 5** depicts a multi-well plate. **FIG. 5A** depicts a well of the multi-well plate holding a sample of co-culture containing a plurality of TCOs and associated FCTs.
**FIG. 6A** is a schematic view of a generalised fluorescent-luminescent fusion protein described herein.
**FIG. 6B** is a schematic view of an exemplary specific fluorescent-luminescent fusion protein described herein.
**FIG. 7A** is an example of kinetic growth measurements of RFP labelled cancers cells and GFP labelled cancer associated fibroblast in a co-culture. **FIG. 7B** and **7C** is an example of the kinetic quantification of the velocity and number of merges of the co-culture treated with Y-27632 as activator of CAFs. **FIG. 7D** is an example of the morphological changes induced by Y-27632 and activation of hPSC21.
**FIG. 8** Plots of cell survival curve (triangles) based on red fluorescence, cell death curve (squares) based on green fluorescence, cell death curve based on luminescence (circles) as a function of cisplatin concentration for spheroids according to Example 2.
**FIG. 9** Plots of cell survival curve (triangles) based on red fluorescence, cell death curve (squares) based on green fluorescence, cell death curve based on luminescence (circles) as a function of cisplatin concentration for organoids according to Example 2.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.*, any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

Provided herein is a screening method for determining an effect of a potential active agent on a sample comprising a co-culture comprising two more different cellular objects (c-objects). One c-object of the two more different c-objects in the co-culture may be a target cellular object, TCO, and another c-object of the two more different c-objects may be a further cell type, FCT. The co-culture may comprise a combination of a TCO and plurality of FCTs. The co-culture provides a therapeutic model for cellular assemblies, for instance, a cancer model, such as patient-derived cancer organoids (TCO) and cancer-associated fibroblasts (FCT). The method is *in vitro.*

In the screening method the TCO has been labelled with a live-cell fluorescent label having a first emission/excitation profile and with a luminescent cell-death marker. Optionally, the FCT has been labelled with a live-cell fluorescent label having a second emission/excitation profile different from the first emission/excitation profile. Where it is an option to have labelled the FCT with a live-cell fluorescent label, it means that the FCT may have been labelled, in which case the second fluorescence image is captured, and subsequent step performed using the second fluorescence image. It in the alternative means that the FCT has not been labelled, in which case the second fluorescence image is not captured, and subsequent step are not performed using the second fluorescence image.

A dataset is captured of the sample, during an acquisition event using a microscope. The dataset comprises a first fluorescent image from the live-cell fluorescent label having the first emission/excitation profile, and a second fluorescent image from the live-cell fluorescent label having the second emission/excitation profile, and a luminescence measurement of the sample.

A plurality of datasets is captured. A one or a plurality of test datasets is captured for a sample contacted with the potential active agent, and one or a plurality of control datasets is captured for a sample not contacted with the potential active agent. Each dataset may be captured at a different time point. Several datasets may be captured at a different time points. Several datasets may be captured at the same time point. An effect of the potential active agent is determined from a difference between the test datasets and control datasets. By difference as used herein, it is meant a change *e.g*. between the test datasets and control datasets, which may or may not include a mathematic subtraction.

The method allows the effect of the active agent to be determined on the TCO alone, for instance, when the FCT has been or has not been fluorescently labelled. The method allows the effect of the active agent to be determined on the TCO and on the FCT independently for instance when both the TCO and FCT have been fluorescently labelled because each can be independently imaged. One or more characterising parameters of the TCO(s) or FCT(s) in the sample (*e.g.* mean, individual or total) can be determined automatically by identification of objects in the image, and automatic measurement of their area, intensity of movements.

A difference of one or more characterising parameters in the absence and presence (usually at varying concentrations) of the potential therapeutic agent allows a determination of an effect of the potential therapeutic agent. The assays may be performed continuously (real time) or at one or more discrete time points. The screening method may be on multiple test samples held on multi-well plates (*e.g*. 384 well). The effect may be therapeutic (*e.g*. cytotoxic, inhibition of growth, cell death), adverse (*e.g.* stimulation of growth, cell proliferation), or non-therapeutic (*e.g.* same effect as control).

The one or plurality of control datasets may be captured for the sample prior to contact with the potential active agent. The one or plurality of control datasets may be captured for a control sample not contacted with the potential active agent, and that is separate from a test sample contacted with the potential active agent.

The method may be used for distinguishing a cytotoxic effect from a cytostatic effect of the potential active agent on the sample. The method may be used for distinguishing a cytotoxic effect from a cytostatic effect of the potential active agent on the TCO in the sample. In a co-culture, labelling the TCO with the luminescent cell-death marker allows accurate monitoring of the TCO cytotoxic/cytostatic status that is not influenced by a cell-death of the FCT. In addition to discerning the TCO cytostatic/cytotoxic response, effects of the potential active agent on the FCT may be measured from its fluorescent image.

The potential active agent may be an anti-cancer (*e.g.* tumour) agent.

The microscope has a fluorescence imaging mode for acquisition of one, two or more fluorescence images of the sample at different emission/excitation profiles, and optionally a Brightfield (BF) imaging mode for acquisition of a Brightfield image of the sample. The images may be a static image, or a part of a moving image (real-time acquisition). The microscope further has a luminescence measurement mode for measurement of luminescence of the sample. The microscope is configured to capture the one or more fluorescence images, and the luminescence and the optional BF image in the acquisition event. An acquisition event is a capture by the microscope of a plurality of images simultaneously or near-simultaneously such the position and orientation of the TCO and optionally of the FCT in the respective images is the same. Typically, the delay between separate captures is in an order of tens of milliseconds. The fluorescence imaging mode operates at a plurality of different wavelengths or channels, in order to capture selectively different fluorescent images at different emission wavelength bands **(*e.g*.** blue, green and red). The microscope is configured to capture an optional Brightfield image, and a first fluorescent image at a first excitation and/or emission wavelength band, and a second fluorescent image at a second excitation and/or emission wavelength band, and optional further fluorescent images at a further excitation and/or emission wavelength bands in the acquisition event. The microscope may be provided with a multi-well plate reading platform that moves a multi-well plate holding a plurality of different samples so a well can be measured by the microscope. Example of a suitable microscope includes the Tecan Spark Cyto.

The TCO and optionally the FCT are each labelled with a distinct live cell fluorescent label that allows the TCO and optionally the FCT be independently fluorescently imaged. Where the TCO alone is fluorescently labelled, one or more characterising parameters of the TCO may be determined of the TCO (but not of the FCO). Where both the TCO and FCT are fluorescently labelled, one or more characterising parameters of the TCO and FCT may be independently determined. The live cell fluorescent label highlights the TCO or optionally the FCT in any state, and also in real-time. It is a universal label. It allows the shape of the TCO and optionally the FCT to be fluorescently imaged. Debris is not labelled and non-cellular objects are not labelled. Examples of live cell fluorescent label include nucleic acid dyes including the Hoechst series of fluorescent dyes (*e.g*. Hoechst 33258 (blue), 34580 (red), 33342 (green)), Nuclight Red (Sartorius), Nuclight Green (Sartorius), Cytolight Green (Sartorius), Cytolight Red (Sartorius), Green fluorescent protein (GFP), Red fluorescent protein (RFP), mCherry (far red).

The TCO is labelled with a live-cell fluorescent label having a first emission/excitation profile, and the optionally the FCT is labelled with a live-cell fluorescent label having a second emission/excitation profile that is different from the first. It allows each label to be imaged separately from the other by changing the excitation wavelength and/or the detection wavelength.

The fluorescent label may be an additive that is added to the TCO or optionally to the FCT, and contact of the additive with the TCO or FCT causes binding and fluorescent labelling of the TCO or FCT; this may be called a transient fluorescent label. Examples include the Hoechst series of fluorescent dyes. To prevent mixing of labels, the TCO or FCT may be labelled prior to addition to the sample.

The fluorescent label may be introduced into the TCO or FCT as a gene (*e.g.* by transfection or transduction of a vector) that expresses a fluorescent label or that expresses a tag that reacts with one or more other substances to produce fluorescence; this may be called a stable fluorescent label. Examples include Red fluorescent protein (RFP), Green fluorescent protein (RFP), mCherry, NucLight/Cytolight Red/Green Lentivirus Reagent (Sartorius).

The luminescent cell-death marker emits luminescence when the cell wall is breached (*e.g.* apoptosis, lysis, cell death) by reaction with one or more reagents in the cell culture medium. The luminescent marker may be introduced into the TCO as a gene (*e.g.* by transfection or transduction of an expression vector) that continually expresses the luminescent cell-death marker.

The luminescent cell-death marker may contain a HiBiT tag (SEQ ID: 1). The HiBiT tag binds with a LgBiT polypeptide in the cell culture medium and there is a release of luminescence upon binding and reaction with a detection reagent in the cell culture medium (*e.g.* Nano-Glo^{®} Endurazine^{™} or Vivazine^{™} Live cell substrate).

Preferably, the luminescent cell-death marker contains a HaloTag-HiBiT fusion protein (SEQ ID: 4) that is a fusion protein comprising a HaloTag polypeptide (SEQ ID: 3) and the HiBiT tag (SEQ ID: 1). A function of the HaloTag polypeptide is to act as a stabilizing fusion protein *e.g.* reducing transmembrane leakage and breakdown of the smaller HiBiT part. Additionally, the HaloTag polypeptide may be used as a ligand for intracellular detection/localization of the HaloTag-HiBiT protein (*e.g.* quantify HaloTag-HiBiT expression for normalization). The HaloTag polypeptide and HiBiT peptide tag may be joined by a linker peptide (HH linker peptide) (*e.g.* SEQ ID: 3).

Examples of expression vectors for HiBiT tag or HaloTag-HiBiT fusion protein include be HaloTag-HiBiT-LentiB3 Transfer Vector CS3055A34 (Promega), or CAG/HaloTag- HiBiT/BlastR Vector CS1956B17 (Promega)

TCO is labelled with the live-cell fluorescent label having a first emission/excitation profile and with the luminescent cell-death marker. The live-cell fluorescent label and the luminescent cell-death marker may be stably expressed within the TCO by translation of a genetic construct encoding a fluorescent-luminescent fusion protein comprising the live-cell fluorescent label and the luminescent cell-death marker.

The live-cell fluorescent label may be any protein that is fluorescent. It is fluorescently active at least within the cell. The live-cell fluorescent label may be a fluorescent protein such as Red fluorescent protein (RFP) (SEQ ID: 5), Green fluorescent protein (GFP) (SEQ ID: 6), or mCherry (SEQ ID: 7).

The luminescent cell-death marker emits luminescence when the cell wall is breached (*e.g.* apoptosis, lysis, cell death) by reaction with one or more reagents in the cell culture medium. The luminescent cell-death marker may contain a HiBiT tag (SEQ ID: 1); it may contain the HaloTag-HiBiT fusion protein (SEQ ID: 4). The HaloTag-HiBiT fusion protein contains the HaloTag polypeptide (SEQ ID: 2) and the HiBiT tag (SEQ ID: 1) joined by a linker peptide (*e.g.* SEQ ID: 3).

The live-cell fluorescent label and the luminescent cell-death marker may be joined by a self-cleaving peptide, that is cleaved within the cell, thereby releasing within the cell the live-cell fluorescent label and the luminescent cell-death marker. The self-cleaving peptide may be T2A (SEQ ID: 8).

Provided, but not currently claimed, is a fluorescent-luminescent fusion protein comprising the live-cell fluorescent label, the luminescent cell-death marker linked by the self-cleaving peptide. Provided is a genetic construct encoding the fluorescent-luminescent fusion protein. The genetic construct can be cloned into a vector (*e.g*. expression vector, replication vector).

By "encoding" is meant that a nucleic acid sequence or part(s) thereof corresponds, by virtue of the genetic code of an organism in question, to a particular amino acid sequence, e.g., the amino acid sequence of a desired polypeptide or protein. By means of example, nucleic acids "encoding" a particular polypeptide or protein may encompass genomic, hnRNA, pre-mRNA, mRNA, cDNA, recombinant or synthetic nucleic acids.

The genetic construct described herein comprises, preferably in that order, a nucleotide sequence encoding a live-cell fluorescent label, a nucleotide sequence encoding a self-cleaving peptide such as a T2A peptide, and a nucleotide sequence encoding a luminescent cell-death marker.

The genetic construct may further comprise a promoter operably linked to the nucleotide sequences encoding the fluorescent-luminescent fusion protein. The term 'operably linked' as used herein refers to the arrangement of various nucleic acid molecule elements relative to each such that the elements are functionally connected and are able to interact with each other. Such elements may include, without limitation, a promoter and/or an enhancer, a transcription terminator, and a coding sequence of a gene of interest to be expressed. The nucleic acid sequence elements, when properly oriented or operably linked, act together to modulate the activity of one another, and ultimately may affect the level of expression of the fluorescent-luminescent fusion protein. By modulate is meant increasing, decreasing, or maintaining the level of activity of a particular element. The position of each element relative to other elements may be expressed in terms of the 5' terminus and the 3' terminus of each element, and the distance between any particular elements may be referenced by the number of intervening nucleotides, or base pairs, between the elements. As understood by the skilled person, operably linked implies functional activity, and is not necessarily related to a natural positional link.

Promoters envisaged in the context of the present invention will be determined by its ability to direct expression in the TCO of interest as can be readily understood by the skilled person. Other sequences may be incorporated in the genetic construct, more particularly the inclusion of sequences which further increase or stabilize the expression of the expressed products (e.g. introns and/or a transcription termination sequence).

In particular embodiments, the expression further comprise a transcription termination sequence. Any polyadenylation signal that directs the synthesis of a polyA tail is useful in the expression cassette described herein, examples of those are well known to one of skill in the art. The genetic construct envisaged herein may be used as such, or typically, they may be part of (i.e. introduced into) a vector.

Provided, but not currently claimed, is an expression vector containing the genetic construct. The expression vector is configured for expression of the genes within a cell (*e.g.* TCO) transfected or transduced with the expression vector.

The vectors disclosed herein may further include an origin of replication that is required for maintenance and/or replication in a specific cell type. One example is when a vector is required to be maintained in a host cell as an episomal genetic element (e.g. plasmid or cosmid molecule). Exemplary origins of replication include, but are not limited to the f1-ori, colE1 ori, and Gram+ bacteria origins of replication.

The vectors taught herein may further contain restriction sites of various types for linearization or fragmentation.

Numerous vectors are known to practitioners skilled in the art and any such vector may be used. Selection of an appropriate vector is a matter of choice. The vector may be a non-viral or viral vector. Non-viral vectors include but are not limited to plasmids, cationic lipids, liposomes, nanoparticles, PEG, PEI, etc. Viral vectors are derived from viruses including but not limited to: retrovirus, lentivirus, adeno- associated virus, adenovirus, herpesvirus, hepatitis virus or the like.

The expression vector may be pLenti-mCherry-T2A-HaloTag-HiBiT transfer vector, pLenti-mCherry-RFP-HaloTag-HiBiT transfer vector, pLenti-GFP-T2A-HaloTag-HiBiT transfer vector.

Methods for transforming a TCO and/or a FCT with a genetic construct or a vector as taught herein above are well known to a skilled person. For example, electroporation, chemical (such as calcium chloride- or lithium acetate-based) transformation methods, microparticles bombardment, glass beads, or viral- or Agrobacterium tumefaciens-mediated transformation methods as known in the art can be used.

The genetic construct or vectors disclosed herein may either be integrated into the nuclear genome of the host cell, in particular the TCO and/or FCT, or they may be maintained in some form (such as a plasmid) extrachromosomally. A stably transformed host cell is one in which the exogenous nucleic acid has become integrated into a chromosome so that it is inherited by daughter cells through chromosome replication.

The TCO is a patient-derived organoid or cancer cell line derived spheroid and the FCT is a fibroblast(s) (more preferably a plurality of fibroblasts) or immune cell(s) (PBMC, NK-cell, Cytotoxic T-cell,...) or endothelial cell. In one combination, TCO is a patient-derived organoid or cancer cell line derived spheroid and the FCT is one or more fibroblasts. In one combination, the TCO is a patient-derived organoid or cancer cell line derived spheroid and the FCT is one or more immune cells (*e.g*. one or more of PBMC, NK-cell, Cytotoxic T-cell,...). In one combination, the TCO is a patient-derived cancer organoid or cancer cell line derived spheroid and the FCT is one or more endothelial cells. In one combination, the TCO is a patient-derived cancer organoid or cancer cell line derived spheroid and the FCT is a combination of fibroblast and/or immune cell and/or endothelial cell.

Alternatively, the FCT is a patient-derived organoid or cancer cell line derived spheroid, and the TCO is a fibroblast(s) (more preferably a plurality of fibroblasts) or immune cell(s) (PBMC, NK-cell, Cytotoxic T-cell,...) or endothelial cell. In one combination, FCT is a patient-derived organoid or cancer cell line derived spheroid and the TCO is one or more fibroblasts. In one combination, the FCT is a patient-derived organoid or cancer cell line derived spheroid and the TCO is one or more immune cells (e.g. one or more of PBMC, NK-cell, Cytotoxic T-cell,...). In one combination, the FCT is a patient-derived cancer organoid or cancer cell line derived spheroid and the TCO is one or more endothelial cells. In one combination, the FCT is a patient-derived cancer organoid or cancer cell line derived spheroid and the TCO is a combination of fibroblast and/or immune cell and/or endothelial cell.

The luminescence of the sample is an indication of cell death of the TCO. Cell death of the TCO is determined (at least) from the luminescence (luminescence intensity) captured in the dataset. The luminescence measurement in the dataset is proportional cell death, in particular to a quantity of cells in the TCO having died. Cell death may be determined from the measured luminescence alone or taking into account a measured quantity of cells in the TCO. The quantity of cells in the TCO may be determined using the first fluorescent image. For example, cell death may be determined or quantified, for each dataset, from the luminescence captured, and one or more of fluorescent area in the first fluorescent image and fluorescent intensity in the first fluorescent image. For example, cell death may be determined or quantified, for each dataset, from:
- a ratio of luminescence and fluorescent area in the first fluorescent image, or
- a ratio of luminescence and fluorescent intensity in the first fluorescent image, or
- a ratio of luminescence and a mean of fluorescent intensity and fluorescent area in the first fluorescent image.

One or more characterising parameters of the TCO and optionally of the FCT from the fluorescent images include one or more static properties (*e.g*. total object count of the TCO, or of the FCT, or of the combined TCO-FCT; mean, total, or individual area of the TCO, or of the FCT, or of the combined TCO-FCT object; mean, total, or individual intensity of the TCO, or of the FCT, or combined of the TCO-FCT object), one or more kinetic properties (*e.g.* mean, total, or individual velocity of the TCO, or of the FCT, or of the combined TCO-FCT object; mean, total, or individual contractility of the TCO, or of the FCT, or of the combined TCO-FCT object; mean, total, or individual splitting of the TCO, or of the FCT, or of the combined TCO-FCT object; mean, total, or individual merging of the TCOs, or of the FCTs, or of the combined TCO-FCT objects). A mean property is a mean for the sample. A total property is a total for the sample. An individual property a property per object in the sample. The characterising parameters can be determined from the brightfield image (BF) image, a first fluorescent image and a second fluorescent image by standard image processing techniques such as connected component analysis.

The screening method described herein may comprise a step of determining an area of the TCO, or of the FCT, or of the combined TCO-FCT object. The effect of the potential active agent is determined from a difference in area of the TCO, or of the FCT, or of the combined TCO-FCT object between the test dataset and control dataset. The effect may be determined at one or at a plurality of time points. The effect of the potential active agent is determined from a difference in area over time of the TCO, or of the FCT, or of the combined TCO-FCT object between the test dataset and control dataset.

The area of the TCO is determined from the first fluorescent images captured at the different time points. The area of the FCT is determined from the second fluorescent images captured at the different time points. The area of the combined TCO-FCT object is determined from the first fluorescent images and second fluorescent images captured at the different time points. The area may be mean (sample), total (sample), or individual area of the TCO, or of the FCT, or of the combined TCO-FCT object.

An increase or decrease in area compared with the control may be indicative that potential active agent has an effect. An increase or decrease in area over time compared with the control may be indicative that potential active agent has an effect.

The screening method described herein may comprise a step of determining an intensity of the TCO, or of the FCT, or of the combined TCO-FCT object. The effect of the potential active agent may be determined from a difference in intensity of the TCO, or of the FCT, or of the combined TCO-FCT object between the test dataset and control dataset. The effect may be determined at one or at a plurality of time points. The effect of the potential active agent may be determined from a difference in intensity over time of the TCO, or of the FCT, or of the combined TCO-FCT object between the test dataset and control dataset.

The intensity of the TCO is determined from the first fluorescent images captured at the different time points. The intensity of the FCT may be determined from the second fluorescent images captured at the different time points. The intensity of the combined TCO-FCT object is determined from the first fluorescent images and second fluorescent images captured at the different time points. The intensity may be mean (sample), total (sample), or individual intensity of the TCO, or of the FCT, or of the combined TCO-FCT object.

An increase or decrease in intensity compared with the control may be indicative that potential active agent has an effect. An increase or decrease in intensity over time compared with the control may be indicative that potential active agent has an effect.

The screening method described herein may comprise a step of determining a luminescence of the sample. In other words, a measurement of luminescent signal intensity in the dataset. The effect of the potential active agent may be determined from a difference in luminescence between the test dataset and control dataset. The effect may be determined at one or at a plurality of time points. The effect of the potential active agent may be determined from a difference in luminescence over time of the sample between the test dataset and control dataset.

An increase or decrease in luminescence compared with the control may be indicative that potential active agent has an effect. An increase or decrease in intensity over time compared with the control may be indicative that the potential active agent has an effect.

The screening method described herein may comprise a step of determining a cell death of the TCO, namely of death of cells forming the TCO. Cell death of the TCO is determined at least from the luminescence captured in the dataset as mentioned elsewhere herein. The effect of the potential active agent may be determined from a difference in cell death of the TCO between the test dataset and control dataset. The effect may be determined at one or at a plurality of time points. The effect of the potential active agent may be determined from a difference over time of the cell death of the TCO between the test dataset and control dataset.

An increase or decrease in cell death of the TCO compared with the control may be indicative that potential active agent has an effect. An increase or decrease in intensity over time compared with the control may be indicative that the potential active agent has an effect.

The screening method described herein may comprise a step of determining a velocity of the TCO, or of the FCT, or of the combined TCO-FCT object. The effect of the potential active agent is determined from a difference in velocity of the TCO, or of the FCT, or of the combined TCO-FCT object between the test dataset and control dataset. The effect may be determined at one or at a plurality of time points. The effect of the potential active agent may be determined from a difference in velocity over time of the sample between the test dataset and control dataset.

The velocity of the TCO is determined from the first fluorescent images captured at the different time points. The velocity of the FCT is determined from the second fluorescent images captured at the different time points. The velocity of the combined TCO-FCT object is determined from the first fluorescent images and second fluorescent images captured at the different time points. The velocity may be mean (sample), total (sample), or individual velocity of the TCO, or of the FCT, or of the combined TCO-FCT object.

An increase or decrease in velocity compared with the control may be indicative that potential active agent has an effect. An increase or decrease in velocity over time compared with the control may be indicative that potential active agent has an effect.

The screening method described herein may comprise a step of determining a contractility of the TCO, or of the FCT, or of the combined TCO-FCT object. The effect of the potential active agent is determined from a difference in contractility of the TCO, or of the FCT, or of the combined TCO-FCT object between the test dataset and control dataset. The effect may be determined at one or at a plurality of time points. The effect of the potential active agent may be determined from a difference in contractility over time of the sample between the test dataset and control dataset.

The contractility of the TCO is determined from the first fluorescent images captured at the different time points. The contractility of the FCT is determined from the second fluorescent images captured at the different time points. The contractility of the combined TCO-FCT object is determined from the first fluorescent images and second fluorescent images captured at the different time points. The contractility may be mean (sample), total (sample), or individual contractility of the TCO, or of the FCT, or of the combined TCO-FCT object.

Contractility is based on tracking of the objects. A mean distance between c-objects (between TCOs, between FCTs, and between TCO and FCT) is measured to determine the amount of spacing between located objects; if the spacing reduces, there is contractility. Alternatively a distance between c-objects (TCOs, FCTs) and the centre of the image is measured to determine the amount of spacing between located objects and the centre. If the spacing reduces, there is contractility towards the center.

An increase or decrease in contractility compared with the control may be indicative that potential active agent has an effect. An increase or decrease in contractility over time compared with the control may be indicative that potential active agent has an effect.

The screening method described herein may comprise a step of determining a splitting of the TCO, or of the FCT, or of the combined TCO-FCT object. The effect of the potential active agent is determined from a difference in splitting of the TCO, or of the FCT, or of the combined TCO-FCT object between the test dataset and control dataset. The effect may be determined at one or at a plurality of time points. The effect of the potential active agent may be determined from a difference in splitting over time of the sample between the test dataset and control dataset.

The splitting of the TCO is determined from the first fluorescent images captured at the different time points. The splitting of the FCT is determined from the second fluorescent images captured at the different time points. The splitting of the combined TCO-FCT object is determined from the first fluorescent images and second fluorescent images captured at the different time points. The splitting may be mean (sample), total (sample), or individual splitting of the TCO, or of the FCT, or of the combined TCO-FCT object.

Splitting is based on tracking of the objects. Splitting may be determined from a branching path of movement of the FCO or of the FCT, or of the combined TCO-FCT object. An increase or decrease in splitting compared with the control may be indicative that potential active agent has an effect. An increase or decrease in splitting over time compared with the control may be indicative that potential active agent has an effect.

The screening method described herein may comprise a step of determining a merging of two or more TCOs, or of two or more FCTs, or of two or more combined TCO-FCT objects. The effect of the potential active agent is determined from a difference in splitting of two or more TCOs, or of two or more FCTs, or of two or more combined TCO-FCT objects between the test dataset and control dataset. The effect may be determined at one or at a plurality of time points. The effect of the potential active agent may be determined from a difference in merging over time of the sample between the test dataset and control dataset.

The merging of the TCO is determined from the first fluorescent images captured at the different time points. The merging of the FCT is determined from the second fluorescent images captured at the different time points. The merging of the combined TCO-FCT object is determined from the first fluorescent images and second fluorescent images captured at the different time points. The merging may be mean (sample), total (sample), or individual splitting of the TCO, or of the FCT, or of the combined TCO-FCT object.

Merging is based on tracking of the objects. Merging may be determined from a converging and joining path of movement of two or more TCOs, or of two or more FCTs, or of two or more combined TCO-FCT objects between the test dataset and control dataset.

An increase or decrease in merging compared with the control may be indicative that potential active agent has an effect. An increase or decrease in merging over time compared with the control may be indicative that potential active agent has an effect.

The screening method may comprise determining a growth of the TCO and FCT as independent objects, and the cell death of the TCO wherein growth is determined from:
- an area of each of the TCO or the FCT determined from the first and second fluorescent images respectively captured in each dataset at different time points, or
- an intensity of each of the TCO or the FCT determined from the first and second fluorescent images respectively captured in each dataset at different time points, or
- a combination of area and intensity of each of the TCO or the FCT determined from the first and second fluorescent images captured in each dataset at the different time points,
wherein cell death of the TCO is determined from (at least) the luminescence captured in each dataset at different time points.

The screening method may comprise determining a growth of the TCO and cell death of the TCO, and optionally determining a growth of the FCT independent of the growth of the TCO, wherein:
- growth of the TCO is determined from:
   - an area of the TCO determined from the first fluorescent image captured in each dataset at different time points, or
   - an intensity of the TCO determined from the first and second fluorescent images respectively captured in each dataset at different time points, or
   - a combination of area and intensity of the TCO determined from the first fluorescent images captured in each dataset at different time points, and
- optionally growth of the FCT is determined from:
   - an area of the FCT determined from the second fluorescent images captured in each dataset, or
   - an intensity of the FCT determined from the second fluorescent images captured in each dataset, or
   - a combination of area and intensity of the FCT determined from the second fluorescent image captured in each dataset,
wherein cell death of the TCO is determined from the luminescence captured in each dataset at the different time points.

The effect of the potential active agent is determined from a difference, between test datasets and control datasets, in growth for the TCO and/or in cell death of the TCO, and/or optionally in growth for the FCT.

A difference that is an increase or decrease in growth of the TCO and/or of the FCT is indicative that the potential active agent has an effect.

A difference that is an increase or decrease in growth of the TCO and/or of the FCT over different time periods is indicative that the potential active agent has an effect.

A difference that is an increase in cell death is indicative that the potential active agent has an effect. The effect is cytotoxic. The effect may be therapeutic.

A difference that is an increase in cell death over different time periods is indicative that the potential active agent has an effect. The effect is cytotoxic. The effect may be therapeutic.

A difference that is a decrease in growth in the absence of cell death is indicative that potential active agent has an effect. The effect is cytostatic. The effect may be therapeutic.

A difference that is a decrease in growth and increase in cell death is indicative that potential active agent has an effect. The effect is cytotoxic. The effect may be therapeutic.

The area may be mean (sample), total (sample), or individual area of the TCO, or of the FCT. The intensity may be mean (sample), total (sample), or individual area of the TCO, or of the FCT.

The screening method may comprise determining interactions between the TCO and FCT as independent objects,
wherein interactions is determined from:
- a merging between the TCO and the FCT determined from the first and second fluorescent images respectively captured in each dataset at different time points, and/or
- a splitting of the TCO and the FCT associated determined from the first and second fluorescent images respectively captured in each dataset at different time points.

The effect of the potential active agent is determined from a difference, between test datasets and control datasets, in the interactions.

A difference that is an increase or decrease in merging between the TCO and the FCT is indicative that the potential active agent has an effect. An example is a merging of an immune cell and organoid/spheroid, or a merging of cancer-associated fibroblast and organoid /spheroid.

A difference that is an increase or decrease in splitting of the TCO from the FCT is indicative that the potential active agent has an effect. An example is a splitting of an immune cell and organoid/spheroid association, or a splitting of a cancer-associated fibroblast and organoid /spheroid association.

A difference that is a decrease in splitting is indicative that potential active agent has an effect. The effect may be therapeutic.

A difference that is a decrease in merging is indicative that potential active agent has an effect. The effect may be therapeutic.

The screening method may comprise determining activity of the TCO and FCT as independent objects, wherein activity is determined from:
- a velocity of the TCO determined from the first fluorescent images captured in each dataset at the different time points, and
- a velocity of the FCT determined from the second fluorescent images captured in each dataset at the different time points.

The effect of the potential active agent is determined from a difference, between test datasets and control datasets, in the activity.

The screening method may comprise determining **activity** of the TCO and optionally activity of the FCT as object independent of the TCO,
wherein
- activity of the TCO is determined from a velocity of the TCO determined from the first fluorescent images captured in each dataset at different time points, and
- optionally activity of the FCO is determined from a velocity of the FCT determined from the second fluorescent images captured in each dataset at different time points.

The effect of the potential active agent is determined from a difference, between test datasets and control datasets, in the activity.

A difference that is an increase or decrease in activity between test datasets and control datasets is indicative that the potential active agent has an effect.

A difference that is a decrease in velocity of the TCO between test datasets and control datasets is indicative that the potential active agent has an effect. The effect may be therapeutic.

A difference that is a decrease in velocity of the FCT between test datasets and control datasets is indicative that the potential active agent has an effect. The effect may be therapeutic.

The characterising parameters of the c-objects *(i.e.* of the TCO and/or of the FCT) in the first fluorescent image (TCO(s)) and second fluorescence image (FCT(s)) may be determined automatically, for instance, using a computer implemented method. The computer implemented method may use standard processing methods for isolating objects in order to calculate their static characterising parameters (area, intensity), and kinetic characterising parameters (velocity, merging, contractility, splitting). Examples of such computer-implemented methods include connected component analysis to isolate individual objects and tracking via edge matching in order to link objects found over time.

C-objects may be identified from a Brightfield (BF) image captured of the sample during the acquisition event, at the same time as the first fluorescent image, the second fluorescent image and the luminescence measurement. Hence, the dataset may further comprise a BF image. The BF image may be processed according to a method disclosed in patent application EP21162619.7 filed on March 15, 2021. Patent application EP21162619.7 is incorporated herein by reference.

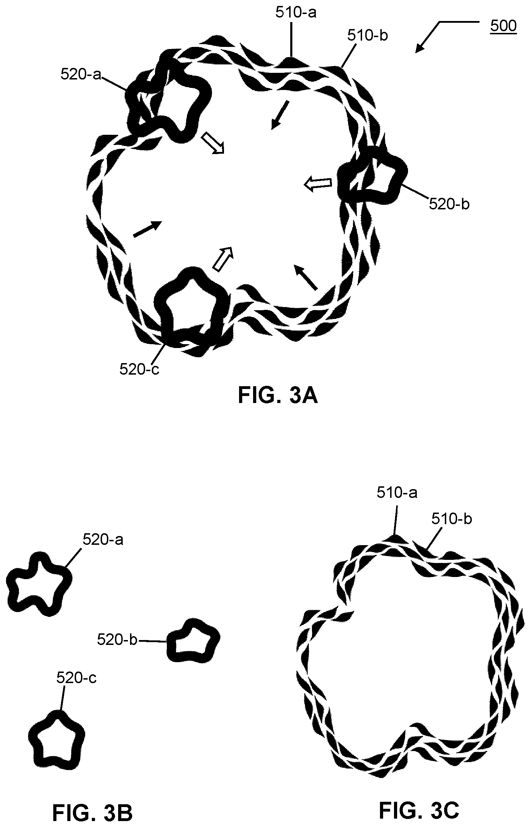

Patent application EP21162619.7 discloses a processing method (200) for highlighting c-objects in a BF image (test BF image (212, 212'). The processing method (200) may be applied to the BF image in order to highlight c-objects (TCOs and or FCOs), which can assist in the identification of c-objects (TCOs and or FCOs) from the first fluorescent image (TCO(s)) and second fluorescence image (FCT(s)). More specifically, the processing method generates an image (primary output image (252)) from the BF image corresponding to the sample as if the TCOs and/or FCOs had been fluorescently labelled. The primary output image can be used to identify cellular objects and consequently the regions in which fluorescence will be measured. Artefacts such as debris, fibres, air bubbles, dust that can sometimes auto-fluoresce may be eliminated artefacts. Patent application EP21162619.7 on page 18, line 30 to page 21, line 14 and **FIG. 3** describes the processing method (200) for generation of the primary output image (252) from a (test) BF image (212, 212'), using a trained predictive model (240).

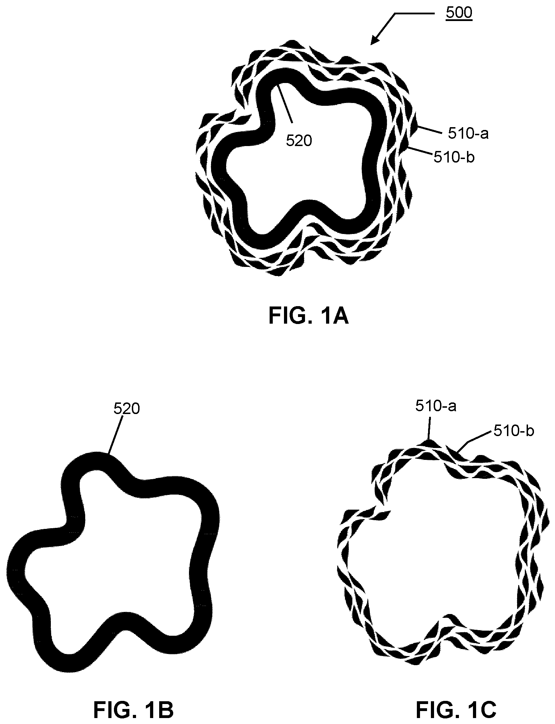

Patent application EP21162619.7 on page 9 line 33 to page 14, line 36 and **FIG. 1** describes a training method (100) for a predictive model (132) that generates the trained predictive model (240). In order to recognise c-objects (TCOs or FCTs) from the BF image, the predictive model is trained using a plurality of training data sets (120).

Training method (100) comprises receiving a plurality of training data sets (120). Each training data set (110) is captured from a training sample (102). Each training data set (110) comprises a training Brightfield (BF) image (112, 112') of the training sample (102) and training c-object data (124) of the training sample (102). The training sample (102) comprises one or more c-objects (TCOs or FCTs) that have each been labelled with a fluorescent label. Both the training BF image (112, 112') and training c-object data (124) are obtained from an acquisition event (110) of a training microscope (104). An acquisition event (110) is a capture by the training microscope (104) of a plurality of images simultaneously or near-simultaneously such the position and orientation of the one or more c-objects in both images is the same. One image of the plurality is the training BF image (112, 112'), and another other image of the plurality is a training fluorescent image (114). Any further training fluorescent images (114) captured during the acquisition event (110) constitute further images in the plurality. The training c-object data (124) is generated automatically from one or more training fluorescent images (114, 114') captured during the acquisition event (110).

At least one of the fluorescent labels in the training sample (102) may be a live cell fluorescent label that highlights only c-objects (all types, or specific types) and in any state.

### Figures

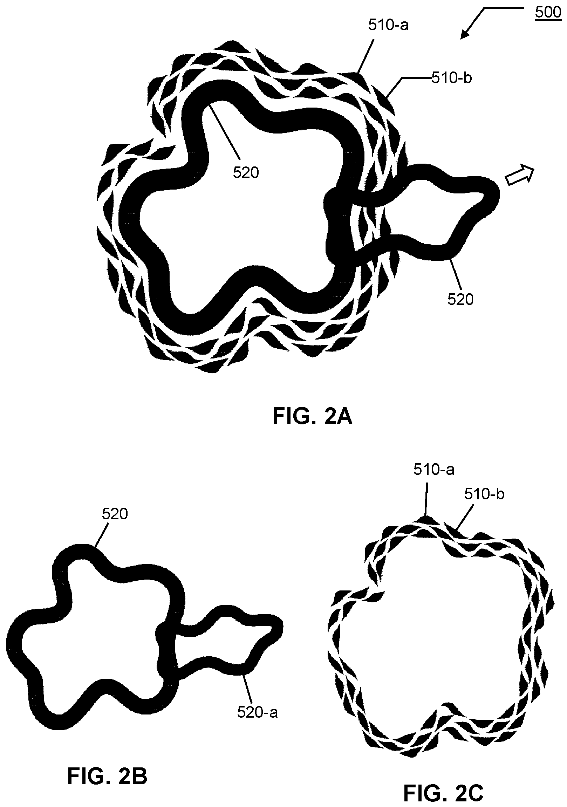

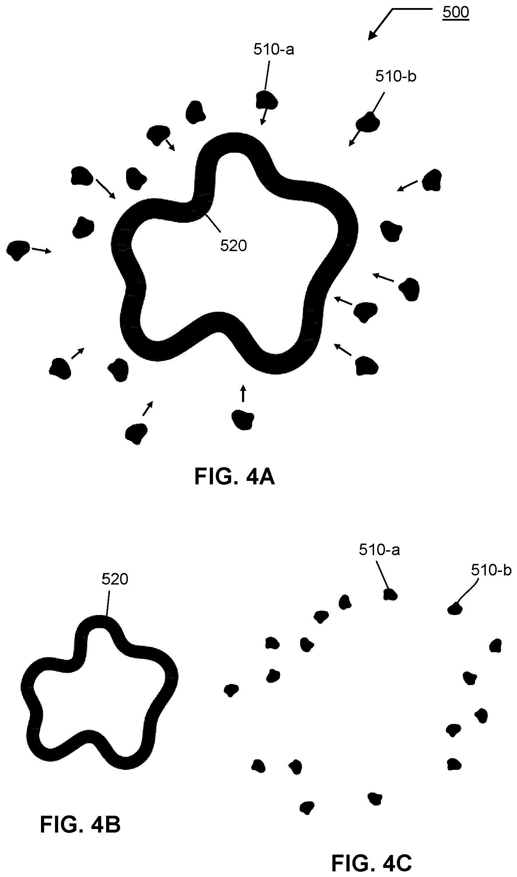

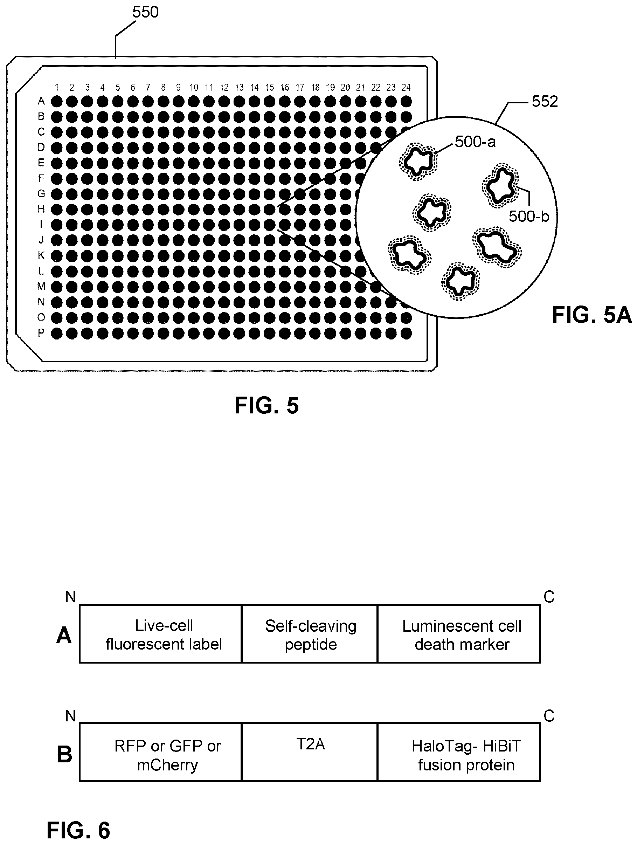

Schematic illustrations of fluorescent images obtained by present method and of different characterising parameters is illustrated in **FIGs. 1A** to **1C****,** **FIGs. 2A** to **2C****,** **FIGs. 3A** to **3C****,** **FIGs. 4A** to **4C****, FIGs. 5A** to **5B.** **FIG. 1A** depicts a first fluorescent image of a TCO (520) that is an organoid superimposed upon a second fluorescent image of associated FCTs (510-a, 510b) that are fibroblasts; **FIGs. 1****B** and **1C** show separately the first and second fluorescent images respectively. **FIG. 2A** depicts a first fluorescent image of an TCO (520) that is an organoid that is splitting superimposed upon a second fluorescent image of associated FCTs (510-a, 510b) that are fibroblasts; **FIGs. 2B** and **2C** show separately the first and second fluorescent images respectively. **FIG. 3A** depicts a first fluorescent image of TCOs (120-a, -b, -c) that are organoids advancing towards each other superimposed upon a second fluorescent image of associated FCTs (510-a, 510b) that are fibroblasts arranged as a contracting ring (contractility); **FIGs. 3B** and **3C** show separately the first and second fluorescent images respectively. **FIG. 4A** depicts a first fluorescent image of a TCO (520) that is an organoid superimposed upon a second fluorescent image of associated FCTs (510-a, 510-b) that are fibroblasts moving towards the organoids (motility); **FIGs. 4B** and **4C** show separately the first and second fluorescent images respectively. **FIG. 5** depicts a multi-well plate (550). **FIG. 5A** depicts a well (552) of the multi-well plate holding a sample of co-culture containing a plurality of TCOs and associated FCTs (500-a, 500-b).

### Sequences

HiBiT tag (SEQ ID: 1): VSGWRLFKKI S
HaloTag polypeptide (SEQ ID: 2): 
MAEIGTGFPF DPHYVEVLGE RMHYVDVGPR DGTPVLFLHG NPTSSYVWRN IIPHVAPTHR
CIAPDLIGMG KSDKPDLGYF FDDHVRFMDA FIEALGLEEV VLVIHDWGSA LGFHWAKRNP
ERVKGIAFME FIRPIPTWDE WPEFARETFQ AFRTTDVGRK LIIDQNVFIE GTLPMGVVRP
 
LTEVEMDHYR EPFLNPVDRE PLWRFPNELP IAGEPANIVA LVEEYMDWLH QSPVPKLLFW
GTPGVLIPPA EAARLAKSLP NCKAVDIGPG LNLLQEDNPD LIGSEIARWL STLEISG
HH linker peptide (SEQ ID: 3): VSQGSSGGGG SGGGGSSG
HaloTag- HiBiT fusion protein (SEQ ID: 4): 
MAEIGTGFPF DPHYVEVLGE RMHYVDVGPR DGTPVLFLHG NPTSSYVWRN IIPHVAPTHR
CIAPDLIGMG KSDKPDLGYF FDDHVRFMDA FIEALGLEEV VLVIHDWGSA LGFHWAKRNP
ERVKGIAFME FIRPIPTWDE WPEFARETFQ AFRTTDVGRK LIIDQNVFIE GTLPMGVVRP
LTEVEMDHYR EPFLNPVDRE PLWRFPNELP IAGEPANIVA LVEEYMDWLH QSPVPKLLFW
GTPGVLIPPA EAARLAKSLP NCKAVDIGPG LNLLQEDNPD LIGSEIARWL STLEISGVSQ
GSSGGGGSGG GGSSGVSGWR LFKKIS
Red fluorescent protein (SEQ ID: 5): 
MRSSKNVIKE FMRFKVRMEG TVNGHEFEIE GEGEGRPYEG HNTVKLKVTK GGPLPFAWDI
LSPQFQYGSK VYVKHPADIP DYKKLSFPEG FKWERVMNFE DGGVVTVTQD SSLQDGCFIY
KVKFIGVNFP SDGPVMQKKT MGWEASTERL YPRDGVLKGE IHKALKLKDG GHYLVEFKSI
YMAKKPVQLP GYYYVDSKLD ITSHNEDYTI VEQYERTEGR HHLFL
Green fluorescent protein (SEQ ID: 6): 
MSKGEELFTG VVPILVELDG DVNGHKFSVS GEGEGDATYG KLTLKFICTT GKLPVPWPTL
VTTFSYGVQC FSRYPDHMKQ HDFFKSAMPE GYVQERTIFF KDDGNYKTRA EVKFEGDTLV
NRIELKGIDF KEDGNILGHK LEYNYNSHNV YIMADKQKNG IKVNFKIRHN IEDGSVQLAD
HYQQNTPIGD GPVLLPDNHY LSTQSALSKD PNEKRDHMVL LEFVTAAGIT HGMDELYK
mCherry (SEQ ID: 7): 
MVSKGEEDNM AIIKEFMRFK VHMEGSVNGH EFEIEGEGEG RPYEGTQTAK LKVTKGGPLP
FAWDILSPQF MYGSKAYVKH PADIPDYLKL SFPEGFKWER VMNFEDGGVV TVTQDSSLQD
GEFIYKVKLR GTNFPSDGPV MQKKTMGWEA SSERMYPEDG ALKGEIKQRL KLKDGGHYDA
EVKTTYKAKK PVQLPGAYNV NIKLDITSHN EDYTIVEQYE RAEGRHSTGG MDELYK
T2A self-cleaving peptide (SEQ ID: 8): EGRGSLLTC GDVEENPGP

A protein mention herein may be a variant of a protein. The term "variant", when used in connection to a protein, for example as in "a variant of protein X", refers to a protein that is altered in its sequence compared to protein X, but that retains the activity of protein X *(i.e.* a functional variant). Preferably, such variant would show at least 80%, more preferably at least 85%, even more preferably at least 90%, and yet more preferably at least 95% such as at least 96%, at least 97%, at least 98% or at least 99% sequence identity to the reference protein, preferably calculated over the entire length of the sequence. The sequence changes may be naturally occurring, for example, due to the degeneracy of the genetic code, or may be introduced artificially, for example by targeted mutagenesis of the respective sequence. Such techniques are well known to the skilled person.

As used herein, the terms "identity" and "identical" and the like are used interchangeably with the terms "homology" and "homologues" and the like herein and refer to the sequence similarity between two polymeric molecules, e.g., between two nucleic acid molecules or polypeptides. Methods for comparing sequences and determining sequence identity are well known in the art. By means of example, percentage of sequence identity refers to a percentage of identical nucleic acids or amino acids between two sequences after alignment of these sequences. Alignments and percentages of identity can be performed and calculated with various different programs and algorithms known in the art. Preferred alignment algorithms include BLAST (Altschul, 1990; available for instance at the NCBI website) and Clustal (reviewed in Chenna, 2003; available for instance at the EBI website). Preferably, BLAST is used to calculate the percentage of identity between two sequences, such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

### Example 1

Pancreatic ductal adenocarcinoma (PDAC) MiaPaca2 cancer cells were transduced with Nuclight Red lentiviral reagent to stably express RFP. Human pancreatic stellate cells 21 (hPSC21) were transduced with the Nuclight Green lentiviral reagent to stably express GFP. Both cell lines were cultures in a 2:1 hPSC21/MiaPaca2 ratio in a 384-well ultra-low attachment plate and treated with the Rho-kinase inhibitor Y-27632, an inducer of a cancer associated fibroblast (CAF) like phenotype of stellate cells (Li et al., Aging, 2020). The cells were incubated at 37°C/5CO2 in the Tecan Spark Cyto multimode plate reader with imaging capabilities and BF, red and green fluorescent images were taken every hour for 48 hours.

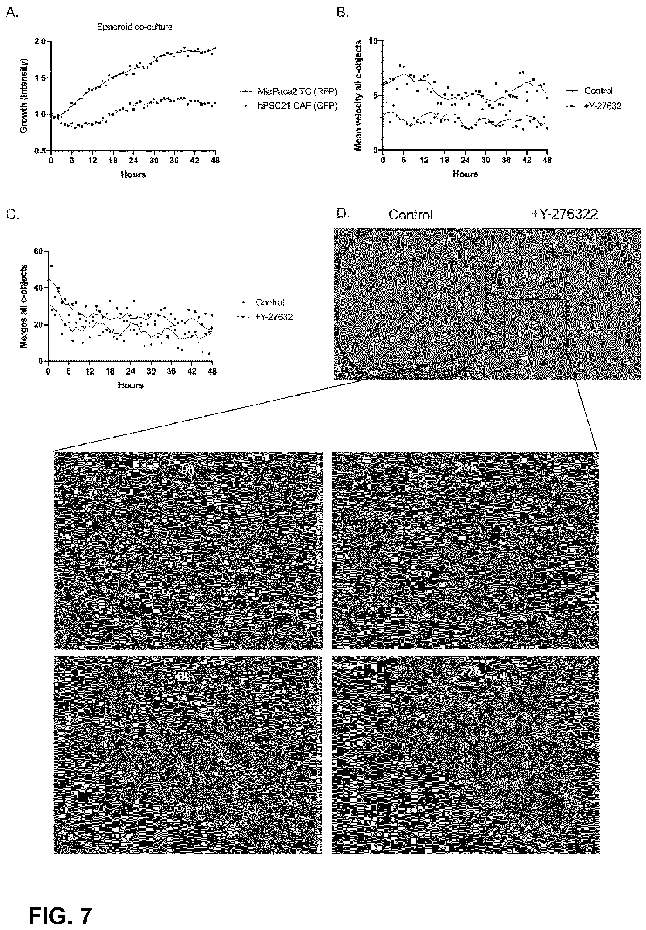

A clear difference in morphology was observed between the control sample and the Y-27632 treated sample with activation and migration of hPSC21 towards the PDAC cancer cells (FIG. 7D). Based on the imaging, the growth of MiaPaca2 and hPSC21 could be independently monitored (FIG. 7A). In addition, increased velocity (FIG. 7B) and number of merges of the objects (FIG. 7C) inside the well related to the activation of hPSC21 by Y-27632 could be quantified.

### Example 2

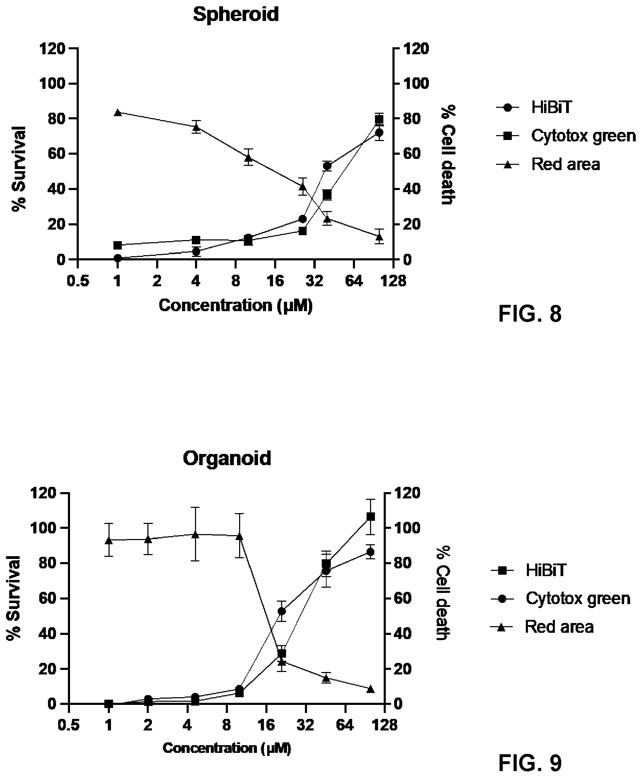

PDAC patient-derived organoids and the PANC1 cancer cell line were transduced with a pLentimCherry-T2A-HaloTag-HiBiT lentiviral vector to express the fluorescent mCherry protein (red) and the luminescent HiBiT-Halotag protein. Cancer cells were grown into organoids or spheroids, plated as 3-day old organoids/spheroids in a 384-well plate and treated with a 6-point titration of cisplatin (1 - 100µM). Cytotox green (Sartorius) was used as a fluorescent cell death marker. At day 5, the Vivazine^{™} Live cell substrate (Promega) was added, and the luminescent signal was measured using a Tecan Spark Cyto, in addition to a whole-well brightfield, green and red fluorescent image at the same timepoint. A survival curve was plotted (left y-axis) based on total red fluorescent area normalised to the vehicle control (100%) and 2µM staurosporine (0%). A cell death curve was plotted based on the overlap of total green area / total brightfield area and normalised to the vehicle control (0%) and 2µM staurosporine (100%). Similarly, a cell death curve was generated based on the luminescent signal normalised to the total red area in each well. Curves are shown in **FIGs. 8** and **9****.** These data show that the survival and cell death of 3D organoids and spheroids can accurately be determined from the combination of red fluorescent imaging and luminescence using the pLenti-mCherry-T2A-HaloTag-HiBiT lentiviral and can therefore be applied in co-cultures.

## Claims

1. A method for determining an effect of a potential active agent on a sample comprising a coculture comprising a target cellular object, TCO, and one or more further cell types, FCT, wherein:
- the TCO has been labelled with a fluorescent live-cell marker having a first emission/excitation profile, and with a luminescent cell-death marker, and
- optionally the FCT has been labelled with a fluorescent live-cell marker having a second emission/excitation profile different from the first emission/excitation profile,
comprising the steps:
- capturing, during an acquisition event using a microscope, a dataset comprising:
- a first fluorescent image from the fluorescent live-cell marker having the first emission/excitation profile, and
- optionally a second fluorescent image from the fluorescent live-cell marker having the second emission/excitation profile, and
- a luminescence measurement of the sample,
wherein a plurality of datasets is captured,
of which one or a plurality of test datasets is captured for a sample contacted with the potential active agent and one or a plurality of control datasets is captured for a sample not contacted with the potential active agent,
- determining a growth of the TCO and cell death of the TCO, and optionally growth of the FCT independent of the growth of the TCO, wherein:
- growth of the TCO is determined from:
- an area of the TCO determined from the first fluorescent image captured in each dataset at different time points, or
- an intensity of the TCO determined from the first and second fluorescent images respectively captured in each dataset at different time points, or
- a combination of area and intensity of the TCO determined from the first fluorescent images captured in each dataset at different time points, and
- cell death of the TCO is determined from the luminescence captured in each dataset at the different time points,
- optionally growth of the FCT is determined from:
- an area of the FCT determined from the second fluorescent images captured in each dataset, or
- an intensity of the FCT determined from the second fluorescent images captured in each dataset, or
- a combination of area and intensity of the FCT determined from the second fluorescent image captured in each dataset,
wherein:
- the effect of the potential active agent is determined from a difference, between test datasets and control datasets, in growth for the TCO and in cell death of the TCO, and optionally in growth for the FCT,
- a difference that is a decrease in growth in the absence of cell death is indicative that potential active agent has a cytostatic effect, and
- a difference that is a decrease in growth and increase in cell death is indicative that potential active agent has a cytotoxic effect, and
wherein:
- the TCO is a patient-derived organoid or cancer cell line derived spheroid, and
- the FCT is a fibroblast(s) or an immune cell(s) such as PBMC, NK-cell, Cytotoxic T-cell, or an endothelial cell(s),
or
- the TCO is a fibroblast(s) or an immune cell(s) such as PBMC, NK-cell, Cytotoxic T-cell, or an endothelial cell(s), and
- the FCT is a patient-derived organoid or cancer cell line derived spheroid,
thereby establishing both cytostatic effect and cytotoxic effect of the potential active agent.

2. The method according to claim 1, further comprising steps of determining interactions between the TCO and FCT as independent objects,
wherein interactions is determined from:
- a merging between the TCO and the FCT determined from the first and second fluorescent images respectively captured in each dataset at different time points, and/or
- a splitting of the TCO and the FCT associated determined from the first and second fluorescent images respectively captured in each dataset at different time points, and
the effect of the potential active agent is determined from a difference, between test datasets and control datasets, in the interactions.

3. The method according to claim 1 or 2, further comprising steps of determining activity of the TCO and optionally activity of the FCT as object independent of the TCO,
wherein
- activity of the TCO is determined from a velocity of the TCO determined from the first fluorescent images captured in each dataset at different time points, and
- optionally activity of the FCO is determined from a velocity of the FCT determined from the second fluorescent images captured in each dataset at different time points, and
the effect of the potential active agent is determined from a difference, between test datasets and control datasets, in the activity.

4. The method according to any one of claims 1 to 3, further comprising a step of determining an area of the TCO, or of the FCT, or of a combined TCO-FCT object from the fluorescence images wherein the effect of the potential active agent is determined from a difference in area of the TCO, or of the FCT, or of the combined TCO-FCT object between the test dataset and control dataset.

5. The method according to any one of claims 1 to 4, further comprising a step of determining an intensity of the TCO, or of the FCT, or of a combined TCO-FCT object from the fluorescence images wherein the effect of the potential active agent is determined from a difference in intensity of the TCO, or of the FCT, or of the combined TCO-FCT object between the test dataset and control dataset.

6. The method according to any one of claims 1 to 5, further comprising a step of determining a velocity of the TCO, or of the FCT, or of a combined TCO-FCT object from the fluorescence images wherein the effect of the potential active agent is determined from a difference in velocity of the TCO, or of the FCT, or of the combined TCO-FCT object between the test dataset and control dataset.

7. The method according to any one of claims 1 to 6, further comprising a step of determining a contractility of the TCO, or of the FCT, or of a combined TCO-FCT object from the fluorescence images wherein the effect of the potential active agent is determined from a difference in contractility of the TCO, or of the FCT, or of the combined TCO-FCT object between the test dataset and control dataset.

8. The method according to any one of claims 1 to 7, further comprising a step of determining a splitting of the TCO, or of the FCT, or of a combined TCO-FCT object from the fluorescence images wherein the effect of the potential active agent is determined from a difference in splitting of the TCO, or of the FCT, or of the combined TCO-FCT object between the test dataset and control dataset.

9. The method according to any one of claims 1 to 8, further comprising a step of determining a merging of the TCO, or of the FCT, or of a combined TCO-FCT object from the fluorescence images wherein the effect of the potential active agent is determined from a difference in merging of the TCO, or of the FCT, or of the combined TCO-FCT object between the test dataset and control dataset.

10. The method according to any one of claims 1 to 9, wherein cell death of the TCO is determined, for each dataset, from the luminescence captured and from a quantity of cells present in the TCO measured from the first fluorescent image.

## Patentansprüche

1. Verfahren zur Bestimmung einer Wirkung eines potentiell wirksamen Mittels auf eine Probe, die eine gemeinsame Kultur eines zellenartigen Zielelements, TCO, und einer oder mehreren weiteren Zellarten, FCT, umfasst, wobei:
- das TCO mit einem Fluoreszenzmarker für lebende Zellen, der ein erstes Emissions-/Anregungsprofil aufweist, und mit einem Lumineszenz-Zelltodmarker markiert wurde, und
- wobei gegebenenfalls die FCT mit einem Fluoreszenzmarker für lebende Zellen markiert wurde, der ein zweites Emissions-/Anregungsprofil aufweist, das sich von dem ersten Emissions-/Anregungsprofil unterscheidet,
wobei es die folgenden Schritte umfasst:
- Erfassen, während eines Aufzeichnungsereignisses unter Verwendung eines Mikroskops, eines Datensatzes, der Folgendes umfasst:
- ein erstes Fluoreszenzbild ausgehend von dem Fluoreszenzmarker für lebende Zellen, welcher das erste Emissions-/Anregungsprofil aufweist, und
- gegebenenfalls ein zweites Fluoreszenzbild ausgehend von dem Fluoreszenzmarker für lebende Zellen, welcher das zweite Emissions-/Anregungsprofil aufweist, und
- eine Lumineszenzmessung der Probe,
wobei eine Mehrzahl an Datensätzen erfasst wird, von denen ein oder mehrere Versuchsdatensätze für eine Probe erfasst wird/werden, welche mit dem potentiell wirksamen Mittel in Kontakt gebracht wurde, und ein oder mehrere Kontrolldatensätze für eine Probe erfasst wird/werden, welche nicht mit dem potentiell wirksamen Mittel in Kontakt gebracht wurde,
- Bestimmen eines Wachstums des TCO und des Zelltods des TCO, und gegebenenfalls des Wachstums der FCT unabhängig vom Wachstum des TCO, wobei:
- das Wachstum des TCO ausgehend von Folgendem bestimmt wird:
- einer Fläche des TCO, die ausgehend von dem ersten Fluoreszenzbild bestimmt wird, welches in jedem der Datensätze zu verschiedenen Zeitpunkten erfasst wurde, oder
- einer Intensität des TCO, die ausgehend von dem ersten und vom zweiten Fluoreszenzbild bestimmt wird, welche in jedem der Datensätze zu verschiedenen Zeitpunkten erfasst wurden, oder
- einer Kombination aus Fläche und Intensität des TCO, die ausgehend von den ersten Fluoreszenzbildern bestimmt wird, welche in jedem der Datensätze zu verschiedenen Zeitpunkten erfasst wurden, und
- der Zelltod des TCO ausgehend von der Lumineszenz bestimmt wird, welche in jedem der Datensätze zu verschiedenen Zeitpunkten erfasst wurde,
- das Wachstum der FCT gegebenenfalls ausgehend von Folgendem bestimmt wird:
- einer Fläche des FCT, die ausgehend von den zweiten Fluoreszenzbildern bestimmt wird, welche in jedem der Datensätze erfasst wurden, oder
- einer Intensität der FCT, die ausgehend von den zweiten Fluoreszenzbildern bestimmt wird, welche in jedem der Datensätze erfasst wurden, oder
- einer Kombination aus Fläche und Intensität der FCT, die ausgehend von dem zweiten Fluoreszenzbild bestimmt wird, welches in jedem der Datensätze erfasst wurde,
wobei:
- die Wirkung des potenziell wirksamen Mittels ausgehend von einem Unterschied zwischen den Versuchsdatensätzen und den Kontrolldatensätzen bestimmt wird, was das Wachstum für das TCO und den Zelltod des TCO sowie gegebenenfalls das Wachstum für die FCT betrifft,
- ein Unterschied, bei dem es sich um eine Abnahme des Wachstums ohne Eintritt eines Zelltods handelt, anzeigt, dass das potenziell wirksame Mittel eine zytostatische Wirkung hat, und
- ein Unterschied, bei dem es sich um eine Abnahme des Wachstums und eine Zunahme des Zelltods handelt, anzeigt, dass das potenziell wirksame Mittel eine zytotoxische Wirkung hat, und
wobei:
- es sich bei dem TCO um ein Organoid, das von einem Patienten stammt, oder ein Spheroid handelt, das von einer Krebszelllinie stammt, und
- es sich bei der FCT um (einen) Fibroblasten oder (eine) Immunzelle(n) wie etwa PBMC, eine NK-Zelle, eine zytotoxische T-Zelle, oder um (eine) Endothelzelle(n) handelt,
oder
- es sich bei dem TCO um (einen) Fibroblasten oder (eine) Immunzelle(n) wie etwa PBMC, eine NK-Zelle, eine zytotoxische T-Zelle, oder um (eine) Endothelzelle(n) handelt, und
- es sich bei der FCT um ein Organoid, das von einem Patienten stammt, oder ein Spheroid handelt, das von einer Krebszelllinie stammt, um auf diese Weise sowohl eine zytostatische Wirkung als auch eine zytotoxische Wirkung des potenziell wirksamen Mittels festzustellen.

2. Verfahren gemäß Anspruch 1, wobei es weiterhin Schritte des Bestimmens von Wechselwirkungen zwischen dem TCO und der FCT als unabhängige Gegenstände umfasst, wobei die Wechselwirkungen ausgehend von Folgendem bestimmt werden:
- einem Verschmelzen des TCO und der FCT, wie es ausgehend von dem ersten beziehungsweise dem zweiten Fluoreszenzbild bestimmt wird, welche in jedem der Datensätze zu verschiedenen Zeitpunkten erfasst wurden, und/oder
- einem Aufspalten des TCO und der zugehörigen FCT, wie es ausgehend von dem ersten beziehungsweise dem zweiten Fluoreszenzbild bestimmt wird, welche in jedem der Datensätze zu verschiedenen Zeitpunkten erfasst wurden, und
die Wirkung des potenziell wirksamen Mittels ausgehend von einem Unterschied zwischen den Versuchsdatensätzen und den Kontrolldatensätzen hinsichtlich der Wechselwirkungen bestimmt wird.

3. Verfahren gemäß Anspruch 1 oder 2, wobei es weiterhin Schritte des Bestimmens der Aktivität des TCO und gegebenenfalls der Aktivität der FCT als vom TCO unabhängiger Gegenstand umfasst,
wobei
- die Aktivität des TCO ausgehend von einer Geschwindigkeit des TCO bestimmt wird, wie sie ausgehend von den ersten Fluoreszenzbildern bestimmt wird, welche in jedem der Datensätze zu verschiedenen Zeitpunkten erfasst wurden, und
- gegebenenfalls die Aktivität der FCO ausgehend einer Geschwindigkeit der FCT bestimmt wird, wie sie ausgehend von den zweiten Fluoreszenzbildern bestimmt wird, welche in jedem der Datensätze zu verschiedenen Zeitpunkten erfasst wurden, und
die Wirkung des potenziell wirksamen Mittels ausgehend von einem Unterschied zwischen den Versuchsdatensätzen und den Kontrolldatensätzen hinsichtlich der Aktivität bestimmt wird.

4. Verfahren gemäß einem beliebigen der Ansprüche 1 bis 3, wobei es weiterhin einen Schritt des Bestimmens einer Fläche des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich der Fläche des TCO, oder der FCT, oder des kombinierten TCO-FCT-Gegenstands, zwischen dem Versuchsdatensatz und dem Kontrolldatensatz bestimmt wird.

5. Verfahren gemäß einem beliebigen der Ansprüche 1 bis 4, wobei es weiterhin einen Schritt des Bestimmens einer Intensität des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich der Intensität des TCO, oder der FCT, oder des kombinierten TCO-FCT-Gegenstands, zwischen dem Versuchsdatensatz und dem Kontrolldatensatz bestimmt wird.

6. Verfahren gemäß einem beliebigen der Ansprüche 1 bis 5, wobei es weiterhin einen Schritt des Bestimmens einer Geschwindigkeit des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich der Geschwindigkeit des TCO, oder der FCT, oder des kombinierten TCO-FCT-Gegenstands, zwischen dem Versuchsdatensatz und dem Kontrolldatensatz bestimmt wird.

7. Verfahren gemäß einem beliebigen der Ansprüche 1 bis 6, wobei es weiterhin einen Schritt des Bestimmens einer Kontraktionsfähogkeit des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich der Kontraktionsfähigkeit des TCO, oder der FCT, oder des kombinierten TCO-FCT-Gegenstands, zwischen dem Versuchsdatensatz und dem Kontrolldatensatz bestimmt wird.

8. Verfahren gemäß einem beliebigen der Ansprüche 1 bis 7, wobei es weiterhin einen Schritt des Bestimmens eines Aufspaltens des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich des Aufspaltens des TCO, oder der FCT, oder des kombinierten TCO-FCT-Gegenstands, zwischen dem Versuchsdatensatz und dem Kontrolldatensatz bestimmt wird.

9. Verfahren gemäß einem beliebigen der Ansprüche 1 bis 8, wobei es weiterhin einen Schritt des Bestimmens eines Verschmelzens des TCO, oder der FCT, oder von einem kombinierten TCO-FCT-Gegenstand, ausgehend von den Fluoreszenzbildern umfasst, wobei die Wirkung des potenziell wirksamen Mittels ausgehend von einem Unterschied hinsichtlich des Verschmelzens des TCO, oder der FCT, oder des kombinierten TCO-FCT-Gegenstands, zwischen dem Versuchsdatensatz und dem Kontrolldatensatz bestimmt wird.

10. Verfahren gemäß einem beliebigen der Ansprüche 1 bis 9, wobei der Zelltod des TCO, für jeden der Datensätze, ausgehend von der erfassten Lumineszenz und von einer Menge an Zellen bestimmt wird, welche anhand einer Messung ausgehend vom ersten Fluoreszenzbild in dem TCO vorliegen.

## Revendications

1. Procédé destiné à déterminer un effet d'un agent actif potentiel sur un échantillon comprenant une coculture comprenant un objet cellulaire cible, TCO, et un ou plusieurs autres types de cellules, FCT, dans lequel :
- le TCO a été marqué avec un marqueur de cellules vivantes fluorescent ayant un premier profil d'émission/excitation, et avec un marqueur de mort cellulaire luminescent, et
- éventuellement, le FCT a été marqué avec un marqueur de cellules vivantes fluorescent ayant un deuxième profil d'émission/excitation différent du premier profil d'émission/excitation,
comprenant les étapes suivantes :
- capture, lors d'un événement d'acquisition au moyen d'un microscope, d'un ensemble de données comprenant :
- une première image fluorescente issue du marqueur de cellules vivantes fluorescent ayant le premier profil d'émission/excitation, et
- éventuellement, une deuxième image fluorescente issue du marqueur de cellules vivantes fluorescent ayant le deuxième profil d'émission/excitation, et
- une mesure de luminescence de l'échantillon,
une pluralité d'ensembles de données étant capturée, dont un ensemble de données d'essai ou une pluralité d'entre eux sont capturés pour un échantillon mis en contact avec l'agent actif potentiel et un ensemble de données de contrôle ou une pluralité d'entre eux sont capturés pour un échantillon non mis en contact avec l'agent actif potentiel,
- détermination de la croissance du TCO et de la mort cellulaire du TCO, et éventuellement de la croissance du FCT indépendamment de la croissance du TCO, dans lequel :
- la croissance du TCO est déterminée à partir :
- d'une surface du TCO déterminée à partir de la première image fluorescente capturée dans chaque ensemble de données à différents points temporels, ou
- d'une intensité du TCO déterminée à partir des première et deuxième images fluorescentes respectivement capturées dans chaque ensemble de données à différents points temporels, ou
- d'une combinaison de la surface et de l'intensité du TCO déterminées à partir des premières images fluorescentes capturées dans chaque ensemble de données à différents points temporels, et
- la mort cellulaire du TCO est déterminée à partir de la luminescence capturée dans chaque ensemble de données aux différents points temporels,
- éventuellement, la croissance du FCT est déterminée à partir :
- d'une surface du FCT déterminée à partir des deuxièmes images fluorescentes capturées dans chaque ensemble de données, ou
- d'une intensité du FCT déterminée à partir des deuxièmes images fluorescentes capturées dans chaque ensemble de données, ou
- d'une combinaison de la surface et de l'intensité du FCT déterminées à partir de la deuxième image fluorescente capturée dans chaque ensemble de données,
dans lequel :
- l'effet de l'agent actif potentiel est déterminé à partir d'une différence de croissance du TCO et de mort cellulaire du TCO, et éventuellement de croissance du FCT, entre les ensembles de données d'essai et les ensembles de données de contrôle,
- une différence qui est une diminution de la croissance en l'absence de mort cellulaire indique que l'agent actif potentiel a un effet cytostatique, et
- une différence qui est une diminution de la croissance et une augmentation de la mort cellulaire indique que l'agent actif potentiel a un effet cytotoxique, et
dans lequel :
- le TCO est un organoïde issu d'un patient ou un sphéroïde issu d'une lignée de cellules cancéreuses, et
- le FCT est un/des fibroblaste(s) ou une/des cellule(s) immunitaire(s) telle(s) que la PBMC, la cellule NK, la cellule T cytotoxique, ou une/des cellule(s) endothéliale(s),
ou
- le TCO est un/des fibroblaste(s) ou une/des cellule(s) immunitaire(s) telle(s) que la PBMC, la cellule NK, la cellule T cytotoxique, ou une/des cellule(s) endothéliale(s),
- le FCT est un organoïde issu d'un patient ou un sphéroïde issu d'une lignée de cellules cancéreuses, ce qui permet d'établir à la fois un effet cytostatique et un effet cytotoxique de l'agent actif potentiel.

2. Procédé selon la revendication 1, comprenant en outre les étapes de détermination d'interactions entre le TCO et le FCT en tant qu'objets indépendants,
les interactions étant déterminées à partir :
- d'une fusion entre le TCO et le FCT déterminée à partir des première et deuxième images fluorescentes respectivement capturées dans chaque ensemble de données à différents points temporels, et/ou
- d'une division du TCO et du FCT associé déterminée à partir des première et deuxième images fluorescentes respectivement capturées dans chaque ensemble de données à différents points temporels, et
l'effet de l'agent actif potentiel est déterminé à partir d'une différence dans les interactions entre les ensembles de données d'essai et les ensembles de données de contrôle.

3. Procédé selon la revendication 1 ou 2, comprenant en outre les étapes de détermination de l'activité du TCO et éventuellement de l'activité du FCT en tant qu'objet indépendant du TCO,
dans lequel
- l'activité du TCO est déterminée à partir d'une vitesse du TCO déterminée à partir des premières images fluorescentes capturées dans chaque ensemble de données à différents points temporels, et
- éventuellement, l'activité du FCO est déterminée à partir d'une vitesse du FCT déterminée à partir des deuxièmes images fluorescentes capturées dans chaque ensemble de données à différents points temporels, et
l'effet de l'agent actif potentiel est déterminé à partir d'une différence d'activité entre les ensembles de données d'essai et les ensembles de données de contrôle.

4. Procédé selon l'une quelconque des revendications 1 à 3, comprenant en outre une étape de détermination d'une surface du TCO, ou du FCT, ou d'un objet TCO-FCT combiné à partir des images de fluorescence, l'effet de l'agent actif potentiel étant déterminé à partir d'une différence de surface du TCO, ou du FCT, ou de l'objet TCO-FCT combiné entre l'ensemble de données d'essai et l'ensemble de données de contrôle.

5. Procédé selon l'une quelconque des revendications 1 à 4, comprenant en outre une étape de détermination d'une intensité du TCO, ou du FCT, ou d'un objet TCO-FCT combiné à partir des images de fluorescence, l'effet de l'agent actif potentiel étant déterminé à partir d'une différence d'intensité du TCO, ou du FCT, ou de l'objet TCO-FCT combiné entre l'ensemble de données d'essai et l'ensemble de données de contrôle.

6. Procédé selon l'une quelconque des revendications 1 à 5, comprenant en outre une étape de détermination d'une vitesse du TCO, ou du FCT, ou d'un objet TCO-FCT combiné à partir des images de fluorescence, l'effet de l'agent actif potentiel étant déterminé à partir d'une différence de vitesse du TCO, ou du FCT, ou de l'objet TCO-FCT combiné entre l'ensemble de données d'essai et l'ensemble de données de contrôle.

7. Procédé selon l'une quelconque des revendications 1 à 6, comprenant en outre une étape de détermination d'une contractilité du TCO, ou du FCT, ou d'un objet TCO-FCT combiné à partir des images de fluorescence, l'effet de l'agent actif potentiel étant déterminé à partir d'une différence de contractilité du TCO, ou du FCT, ou de l'objet TCO-FCT combiné entre l'ensemble de données d'essai et l'ensemble de données de contrôle.

8. Procédé selon l'une quelconque des revendications 1 à 7, comprenant en outre une étape de détermination d'une division du TCO, ou du FCT, ou d'un objet TCO-FCT combiné à partir des images de fluorescence, l'effet de l'agent actif potentiel étant déterminé à partir d'une différence de division du TCO, ou du FCT, ou de l'objet TCO-FCT combiné entre l'ensemble de données d'essai et l'ensemble de données de contrôle.

9. Procédé selon l'une quelconque des revendications 1 à 8, comprenant en outre une étape de détermination d'une fusion du TCO, ou du FCT, ou d'un objet TCO-FCT combiné à partir des images de fluorescence, l'effet de l'agent actif potentiel étant déterminé à partir d'une différence de fusion du TCO, ou du FCT, ou de l'objet TCO-FCT combiné entre l'ensemble de données d'essai et l'ensemble de données de contrôle.

10. Procédé selon l'une quelconque des revendications 1 à 9, dans lequel la mort cellulaire du TCO est déterminée, pour chaque ensemble de données, à partir de la luminescence capturée et à partir d'une quantité de cellules présentes dans le TCO mesurée à partir de la première image fluorescente.
